(19) Europäisches Patentamt
European Patent Office
Office européen des brevets

(11) EP 1 704 870 A1

(12) EUROPEAN PATENT APPLICATION

(43) Date of publication:
**27.09.2006 Bulletin 2006/39**

(21) Application number: **06001402.4**

(22) Date of filing: **24.01.2006**

(51) Int Cl.:
*A61K 41/00* (2006.01) *A61K 47/48* (2006.01)
*A61K 31/409* (2006.01) *A61P 35/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **25.03.2005 JP 2005089865**

(71) Applicant: **NIPRO CORPORATION**
**Kita-ku, Osaka-shi, Osaka-fu,**
**531-8510 (JP)**

(72) Inventors:
- **Hirotsu, Ichiro**
  **NIPRO CORPORATION**
  **Osaka-shi**
  **Osaka-fu, 531-8510 (JP)**
- **Ohkawa, Yuki**
  **NIPRO CORPORATION**
  **Osaka-shi**
  **Osaka-fu, 531-8510 (JP)**
- **Yamamoto, Hisashi**
  **NIPRO CORPORATION**
  **Osaka-shi**
  **Osaka-fu, 531-8510 (JP)**
- **Sato, Makoto**
  **NIPRO CORPORATION**
  **Osaka-shi**
  **Osaka-fu, 531-8510 (JP)**
- **Kobayashi, Koichi**
  **NIPRO CORPORATION**
  **Osaka-shi**
  **Osaka-fu, 531-8510 (JP)**
- **Horinouchi, Hirohisa**
  **NIPRO CORPORATION**
  **Osaka-shi**
  **Osaka-fu, 531-8510 (JP)**

(74) Representative: **Albrecht, Thomas et al**
**Kraus & Weisert**
**Patent- und Rechtsanwälte**
**Thomas-Wimmer-Ring 15**
**80539 München (DE)**

(54) **Use of iron compounds as radiosensitizers**

(57) There is provided a radiosensitizer containing an iron compound, more specifically, a radiosensitizer containing an iron compound selected from an inorganoiron compound, such as iron chloride, iron oxide, iron hydroxide, and iron sulfate; and an organoiron compound, such as saccharated ferric oxide, iron citrate, iron gluconate, chondroitin sulfate/iron colloid, cideferron, ferrotrenine, iron fumarate, iron pyrophosphate, a porphyrin-iron complex, and an albumin incorporating porphyrin-iron complex.

EP 1 704 870 A1

## Description

TECHNICAL FIELD OF THE INVENTION

The present invention relates to a radiosensitizer for improving a response rate of radiation therapy for malignant tumors.

BACKGROUND ART OF THE INVENTION

Conventionally, as therapies for malignant tumors, for example, leukemia, lymphomatosis, carcinoma or sarcoma, there have been performed radiotherapy as well as surgical therapy, chemical therapy, immunotherapy, thermotherapy, and the like. Of these, radiotherapy achieves a high local control rate in many types of malignant tumors and is now used as an effective therapeutic method. With the recent rise of radiochemistry and radiobiology, an agent to enhance an effect of radiation has come to be used concomitantly for a treatment of a radioresistant tumor. In treating a cancer by radiation, the presence of a cell having a resistance to radiation deteriorates a response rate of radiation therapy and causes cancer recurrence.

Radiotherapy is highly dependent on selection of an agent to synergistically increase a biological effect of radiation, and many reports on developments of radiosensitizers have been made in recent years. For example, there have been reported texaphyrins as a radiosensitizer (Patent Document 1), a radiosensitizer containing a chlorin e6 (Patent Document 2), a radiosensitizer containing a nitro-5-deazaflavin derivative (Patent Document 3), a hypoxic cell radiosensitizer containing 2-nitroimidazole derivative as an active ingredient (Patent Document 4), radiosensitizers containing boronated metaroporphyrins (Patent Document 5) and the like.

Patent Document 1 WO 95/10307
Patent Document 2 JP 05-194268A
Patent Document 3 JP 2000-212087A
Patent Document 4 JP 06-298739A
Patent Document 5 WO 02/098417

In radiotherapies for cancer in recent years, a hypoxic cancer cell, which is generated from a cancer cell in which the rate of tumor proliferation becomes higher than that of blood vessel growth, has a resistance to radiation twice to three-times as high as that of a normal pressure oxygen cancer cell, which causes a decrease in a cure rate or recurrence. It is desired to develop a cell radiosensitizer that synergistically increases a biological effect of radiation, has no side effects such as expression of neurotoxicity due to accumulation, and is infinitely safe.

SUMMARY OF THE INVENTION

The inventors of the present invention have made studies on an agent having a strong effect to increase sensitivity to radiation, that is, having a radiosensitizing effect on a cancer cell. As a result, they have surprisingly found out that a heme compound (ferroporphyrin or ferroheme, ferriporphyrin or ferriheme) has a high affinity to a cancer cell and has a radiosensitizing effect. In addition, they have found out that a compound having an iron element in its molecule has a radiosensitizing effect. The inventors of the present invention have made various studies based on these findings and as a result have completed the present invention.

That is, the present invention relates to:

(1) a radiosensitizer which comprises an iron compound;
(2) the radiosensitizer according to the above item (1), wherein the iron compound is an inorganoiron compound, or an organoiron compound selected from the group consisting of saccharated ferric oxide, iron citrate, iron acetate, iron gluconate, chondroitin sulfate/iron colloid, cideferron, ferrotrenine, iron fumarate, iron pyrophosphate, a porphyrin-iron complex, and an albumin incorporating porphyrin-iron complex;
(3) the radiosensitizer according to the above item (2), wherein the inorganoiron compound is selected from the group consisting of iron chloride, iron oxide, iron hydroxide, and iron sulfate;
(4) the radiosensitizer according to the above item (1), wherein the iron compound is an organoiron compound selected from the group consisting of saccharated ferric oxide, iron citrate, iron gluconate, chondroitin sulfate/iron colloid, cideferron, ferrotrenine, iron fumarate, iron pyrophosphate, a porphyrin-iron complex, and an albumin incorporating porphyrin-iron complex;
(5) the radiosensitizer according to the above item (1), wherein the iron compound is saccharated ferric oxide, chondroitin sulfate/iron colloid, a porphyrin-iron complex, or an albumin incorporating porphyrin-iron complex;

(6) the radiosensitizer according to the above item (4) or (5), wherein iron in the porphyrin-iron complex is Fe(II);
(7) the radiosensitizer according to the above item (4) or (5), wherein the porphyrin-iron complex has not a phenyl substituent substituted by a substituent having a boron atom on the porphyrin ring of the porphyrin-iron complex;
(8) the radiosensitizer according to the above item (4) or (5), wherein the porphyrin-iron complex is a non-boronated porphyrin-iron complex;
(9) the radiosensitizer according to the above item (1), wherein the iron compound is an albumin incorporating porphyrin-iron complex;
(10) the radiosensitizer according to the above item (9), wherein the albumin is recombinant albumin;
(11) a kit for treatment of cancer containing an iron compound as a radiosensitizer, and a chemotherapeutic agent;
(12) the kit according to the above item (11), wherein the radiosensitizer and the chemotherapeutic agent are separately packaged;
(13) the kit according to the above item (11), characterized in that the radiosensitizer as defined in the above item (1) is administered within one hour after a chemotherapeutic agent is administered, followed by radiation treatment;
(14) use of an iron compound as defined in the above items (1) to (10) for the preparation of a radiosensitizer and a kit for treatment of cancer containing said iron compound as a radiosensitizer, and a chemotherapeutic agent;
(15) the use according to the above item (14), wherein the radiosensitizer and the chemotherapeutic agent are separately packaged; and
(16) the use according to the above item (14), characterized in that the radiosensitizer as defined in the above item (1) is administered within one hour after a chemotherapeutic agent is administered, followed by radiation treatment.

## EFFECTS OF THE INVENTION

The radiosensitizer of the present invention is infinitely safe, has an excellent effect to enhance radiosensitivity of a hypoxic cancer cell that is resistant to radiotherapy, and is useful as a radiosensitizer in radiotherapy for various malignant tumors. The radiosensitizer of the present invention is an excellent agent because it has high affinity to an electron and also has high affinity to a cancer cell.

## BRIEF DESCRIPTION OF THE DRAWINGS

Fig. 1 A graph showing comparison of rat survival times in the cases of: (1) combined use of recombinant human serum albumin incorporating porphyrin-iron complex (rHSA-FecycP) and X-rays, (2) combined use of saccharated ferric oxide and X-rays, and (3) combined use of chondroitin sulfate/iron colloid and X-rays; and as controls, (4) non-treatment, (5) only administration of rHSA-FecycP, (6) only X-rays irradiation, and (7) combined use of rHSA and X-rays.
Fig. 2 A graph showing comparison of growth delay effects on rat tumors in the cases of: (1) combined use of rHSA-FecycP and X-rays, and (2) combined use of saccharated ferric oxide and X-rays, and (3) combined use of chondroitin sulfate/iron colloid and X-rays; and as controls, (4) non-treatment, (5) only administration of rHSA-FecycP, (6) only X-rays irradiation, and (7) combined use of rHSA and X-rays.
Fig. 3 A graph showing comparison of growth delay effects on rat tumors in the cases of: (1) combined use of met-oxidized rHSA-FecycP (Met-rHSA-FecycP) and X-rays; and as controls, (2) non-treatment, (3) only administration of Met-rHSA-FecycP, and (4) combined use of rHSA administration and X-rays.

## DETAILED DESCRIPTION OF THE INVENTION

A radiosensitizer generally enhancesradiosensitivity at cell level but has no anticancer function in itself, so that the agent itself is not referred to as an anticancer agent.
Examples of the iron compound to be used as the radiosensitizer of the present invention include an inorganic or organic compound having an iron ion (Fe(II) or Fe(III)) in its molecule.
Examples of the inorganic compound containing an iron ion include iron chloride, iron bromide, iron iodide, iron oxide, iron hydroxide, iron sulfate, iron nitrate, yellow iron oxide, yellow iron sesquioxide, black iron oxide, iron sesquioxide, and a chelate compound of a complex ion (for example, halogeno, cyano, thiocyanate, oxalato, etc.) with an iron ion.
Examples of the organic compound having an iron (Fe) ion in its molecule include saccharated ferric oxide, iron citrate, iron acetate, iron gluconate, chondroitin sulfate/iron colloid, cideferron, ferrotrenine, iron fumarate, iron pyrophosphate, a porphyrin-iron complex, and an albumin incorporating porphyrin-iron complex. Of these, saccharated ferric oxide, chondroitin sulfate/iron colloid, a porphyrin-iron complex, and an albumin incorporating porphyrin-iron complex are preferable. An albumin incorporating porphyrin-iron complex is particularly preferable. The porphyrin of the

porphyrin-iron complex and albumin incorporating porphyrin-iron complex is preferably a non-boronated porphyrin.

A macrocyclic tetrapyrrole ring, which is a basic skeleton of a porphyrin, is represented by the following formula (with numbering). Porphyrin is a macrocyclic compound in which four pyrrole rings are alternately linked with four methine groups at the α-positions or a derivative thereof.

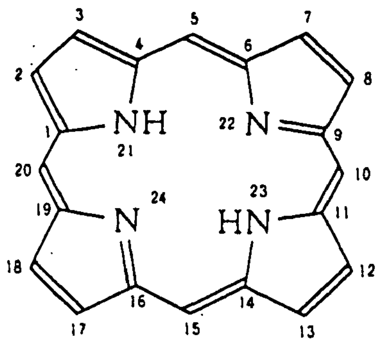

Specific examples of the porphyrin-iron complex include a compound having the general formula (1):

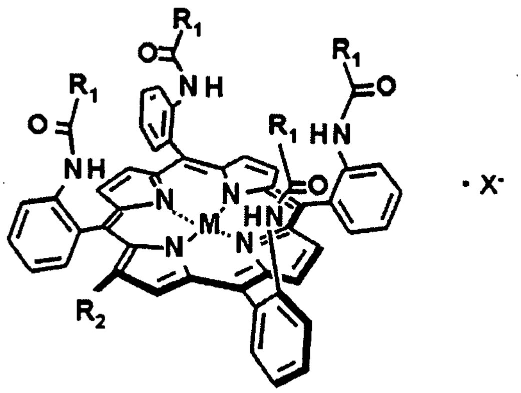

(1)

wherein $R_1$ represents a 1-(saturated chain-hydrocarbon residue)-1-alicyclic group such as a 1-methyl-l-cyclohexyl group, $R_2$ represents a substituent, M represents a Fe ion, $X^-$ represents ahalogenion, and the number of $X^-$ is a number calculated by subtracting 2 from the valence of the Fe ion.

The compound having the general formula (1) above is preferably a compound having the general formula (2):

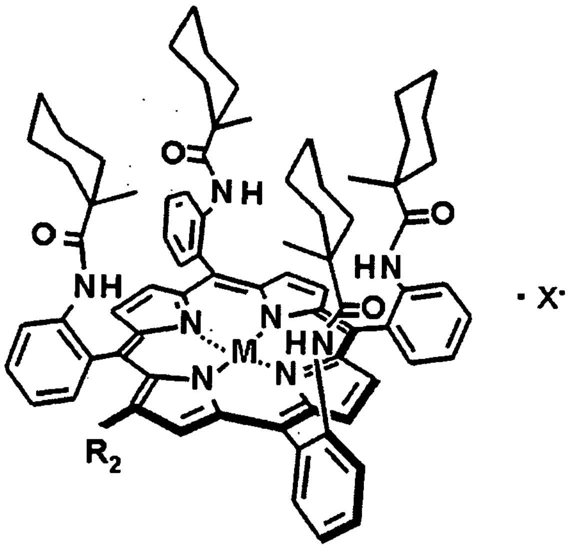

(2)

wherein the symbols in the formula have the same meanings as defined above.

In the general formula (1) above, $R_2$ represents a group having the general formula (3):

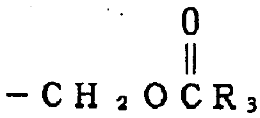

(3)

wherein $R_3$ represents a $C_1$-$C_{18}$ alkyl group, or a group having the general formula (4):

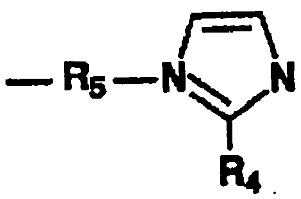

(4)

wherein $R_4$ represents a group that does not inhibit coordination of imidazole linked therewith to the central metal iron, and $R_5$ represents an alkylene group.

In the general formula (4) above, $R_4$ is preferably hydrogen or a $C_1$-$C_3$ alkyl group, and $R_5$ is preferably a $C_1$-$C_{10}$ alkylene group.

Among tetraphenylporphyrin-iron complexes having the general formula (1), preferable is a complex in which Fe that is the central metal (M) is in a divalent state and one base is coordinated. The complex has the general formula (5) or the general formula (6).

The general formula (5):

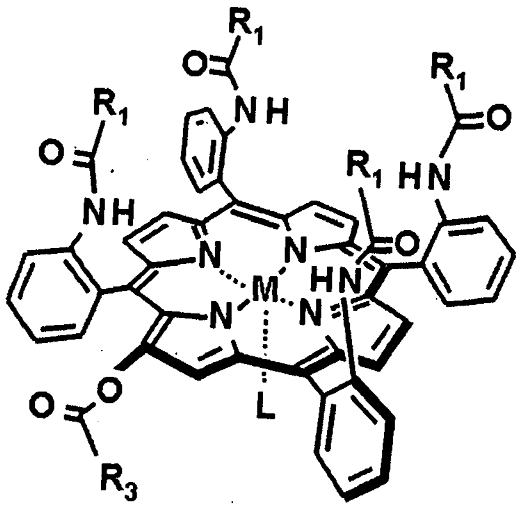

(5)

wherein L represents a base, e.g., a nitrogen axial ligand of an imidazole derivative, and the other symbols have the same meanings as defined above

The general formula (6):

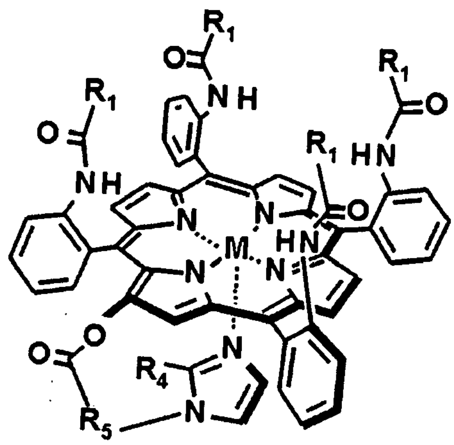

(6)

wherein the symbols in the formula have the same meanings as defined above.

For the porphyrin-iron complex, a tetraphenylporphyrin-iron complex having the general formula (7) is particularly preferable.

The general formula (7):

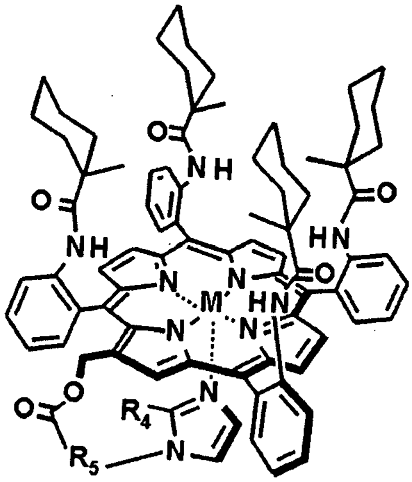

(7)

wherein M represents an iron ion, $R_4$ represents a group that does not inhibit coordination of a nitrogen atom in an imidazole ring linked therewith to the central metal iron, and $R_5$ represents an alkylene group.

Among porphyrin-iron complexes having the general formula (7) above, particularly preferable is a 2-[8-(2-methyl-1-imidazolyl)octanoyloxymethyl]-5,10,15,20-tetrakis{[α,α,α,α-o-(1-methylcyclohexanoylamino)]phenyl}-porphinatoiron complex having the formula (8).

There are used porphyrin-iron complexes having the formula (8):

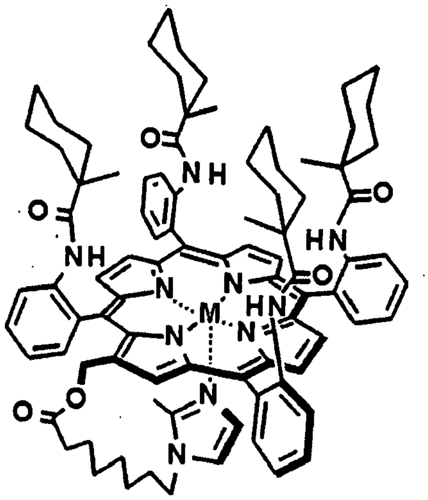

(8)

wherein M represents an iron ion; and
the formula (9):

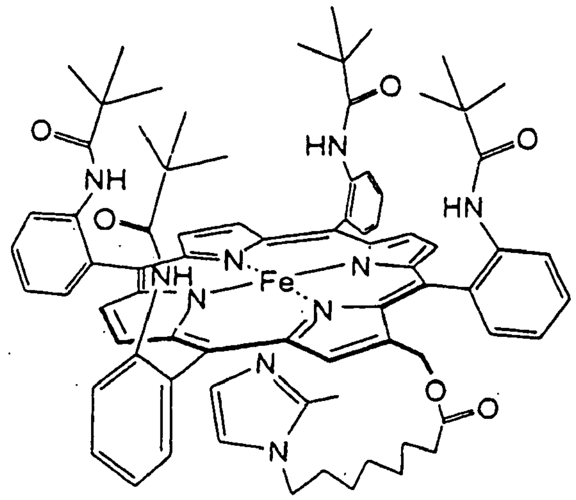

(9)

,known porphyrin derivatives present in animals and plants, for example, etioporphyrin, mesoporphyrin, protoporphyrin, deuteroporphyrin, hematoporphyrin, coproporphyrin, uroporphyrin, tetrabenzoporphyrin, dibenzo[b,1]porphyrin, cyclopenta[at]porphyrin, an iron complex with 3H-benzo[at]porphyrin or the like, a porphyrin-iron complex described in JP 2004-277329A, a porphyrin-iron complex described in JP 2004-10495A, and a porphyrin-iron complex described in JP 06-271577A.

The porphyrin-iron complex albumin inclusion compound, that is, an inclusion compound of the above-described porphyrin-iron complex with albumin is made of a porphyrin-iron complex embedded/fixed/included in an internal hydrophobic region formed by albumin. The number of the porphyrin-iron complex (es) to be linked with one mol of albumin (forexample, human serumalbumin) is generally about 1 to 8. The number of the porphyrin-iron complex (es) included in/linked with one mol of albumin may be determined by creating a Scatchard plot (see C. J. Halfman, T. Nishida, Biochemistry vol. 11, pp.3493 (1972)) or the like.

The porphyrin-iron complex to be used in the present invention can be easily produced by a known method or a method known per se, for example, production methods described in JP 06-271577A, JP 2004-277329A, JP 2004-10495A, or the like.

For example, the tetraphenylporphyrin-iron complex having the general formula (1) can be produced in a manner similar to a production method of a 2-[8-(2-methyl-l-imidazolyl)-octanoyloxymethyl]-5,10,15,20-tetrakis{[α,α,α,α-o-(1-methyl-cyclohexanoylamino)]phenyl}porphinatoiron complex having the formula (8) (T.Komatsu et al., Bioconjugate Chemistry vol.13, pp.397-402 (2002)).

The inclusion compound of a porphyrin-iron complex with albumin can be produced by a known method (T.Komatsu et al., Bioconjugate Chemistry vol.13, pp.397-402 (2002)) or a method known per se. Moreover, the inclusion compound can be produced by, for example, the following method.

First, a porphyrin-iron complex is dissolved in a solvent, (e.g., ethanol), and an aqueous solution (as a solvent, e.g., water, phosphate buffer (pH 5 to 9), physiological saline, Krebs-Ringer's solution and so on) of albumin, for example, human serum albumin, is added thereto, followed by mild shaking. The resultant water dispersion is concentrated to about 10% of the total volume by ultrafiltration (for example, an ultrafiltration membrane with a molecular weight cut off of 20,000 to 40,000 is used). A solvent, e.g., water, phosphate buffer (pH 5 to 9), physiological saline, Krebs-Ringer's solution and so on is added again to the resultant, followed by concentration by ultrafiltration. The process of adding a solvent, followed by concentration by ultrafiltration is repeated until the ethanol concentration in the dispersion liquid is not more than 100 ppm, to thereby yield an albumin incorporating porphyrin-iron complex. The dispersion liquid causes no precipitation, aggregation and so on, even after preservation for several months at 4 to 35°C and is very stable.

In the porphyrin-iron complex, the central iron can be reduced from trivalent to divalent by a conventional method such as by addition of a reducing agent (e.g., an aqueous solution of sodium dithionite, ascorbic acid and so on).

The reduction reaction can be performed not only by addition of a reducing agent but also by using palladium carbon/hydrogen gas. For example, the central iron can be reduced by: dissolving a porphyrin-iron (III) complex in dry dichloromethane, benzene, toluene, or the like; adding a small amount of palladium carbon: and then thoroughly blowing hydrogen gas therethrough at room temperature. After the reduction reaction, palladium carbon is separated and removed by filtration, and the thus-obtained filtrate is dried under vacuum to be subjected to the further reactions.

The above-described reduction is preferably performed before an inclusion reaction of albumin.

The albumin to be used in the present invention may be human serum albumin, recombinant human serum

albumin, bovine serum albumin, or the like, and its origin is not limited. In application to human, human serum albumin or human serum albumin produced by a gene recombination technique (hereinafter, referred to as recombinant human serum albumin) is preferable and, particularly, recombinant human serum albumin is more preferable.

In recent years, with the development of gene recombination techniques, there has been developed high-purity recombinant human serum albumin having exactly the same structure/composition and physicochemical characteristics as those of human serum albumin (see K. Kobayashi et al., Therapeutic Apheresis, 2(4), pp.257-262 (1998)). A porphyrin-iron complex-recombinant human serum albumin inclusion compound, which is formed by inclusion of a substituted porphyrin-iron complex in the recombinant human serum albumin, may be provided as a total synthesis system, so that it is relatively easily produced on an industrial scale.

In radiotherapy, DNA of a cancer cell is damaged not only by radiation directly but also by generation of reactive oxygen species such as hydroxy radicals. Iron catalyzes a Fenton reaction to generate hydroxy radicals, so that the catalytic effect provides an excellent radiosensitizing effect.

The iron compound to be used in a preparation of the present invention may be formed into various dosage forms by a general method, such as an injection, suppository, tablet, powder, granule, capsule, pill, ointment, liquid, patch, cataplasm, and aerosol. In the case of producing an injection, an appropriate solvent and, if necessary, a pH regulator,buffer,stabilizer,suspension,solubilizer,carrier, or the like are added to prepare an injection by the conventional method.

Examples of the stabilizer to be used include sodium sulfite and sodiummetasulfite. Examples of the suspension to be used include methylcellulose, polysorbate 80, and gum arabic. Examples of the solubilizer include polyoxyethylene hardened castor oil, nicotinamide, and polyoxyethylene sorbitan monolaurate.

An injection can be produced by, for example: preliminarily dissolving, dispersing, or emulsifying an iron compound in an aqueous carrier such as saline for injection; or forming an iron compound into powder for injection and dissolving, dispersing, or emulsifying the powder before use.

In the case of using an albumin incorporating porphyrin-iron complex as an iron compound, the compound may be used after being dispersed in a physiologically acceptable aqueous medium, for example, saline, such as phosphate buffered saline.

Examples of an administration method for an injection include intravenous administration, intra-arterial administration, intraportal administration, intramuscular administration, intraperitoneal administration, subcutaneous administration, and direct intralesional administration.

A solid preparation such as a tablet, powder, granule, capsule, pill, or suppository, and a liquid preparation such as syrup can be produced by adding a filler, binder, disintegrator, lubricant, colorant, seasoning, flavor, extender, or coating agent to an iron compound according to a conventional method. For example, in the case of using a porphyrin-iron complex as an iron compound, the compound may be formulated into the tablet, powder, granule, capsule, pill or syrup according to a conventional method, and may be administered orally.

The ointment, liquid, patch, cataplasm, or aerosol can be produced from an iron compound and an appropriate carrier by a conventional method and may be directly applied as an external preparation to a lesion site. The radiosensitizers of the present invention may be used singly or in a combination of two or more.

The radiation treatment using a radiosensitizer comprising an iron compound is generally conducted after administration of chemotherapeutical agents to reduce cancer cells. Radiosensitizers of the present invention may be in a kit combined with chemotherapeutic agents.

In case of a kit preparation, chemotherapeutic preparations and radiosensitizers contained in the kit may be for parenteral administration, or one of them may be for parenteral administration, and the other may be for oral administration, or both may be for oral administration. As chemotherapeutic agents, carcinostatic(anticancer)agentswhich are generally used for cancer treatment include, for example, antitumor allkylating agents such as cyclophosphamide, ifosfamide and melphalan; antitumor antimetabollites such as methotrexate, gemcitabine and 5-fluorouracil(5-FU); antitumor antibiotics such as actinomycin D (dactinomycin), doxorubicin(adriamycin), daunorubicin (daunomycin) and epirubicin; plant-derived antitumor agents such as vincristine, etoposide, docetaxel and paclitaxel; camptothecin derivatives such as camptothecin; platinum-complex compounds such ascisplatin,carboplatin and oxaliplatin;antitumor tyrosine kinase inhibitors such as gefitinib; and biological response modifiers such as krestin and picibanil. These anti-tumor agents may be administered according to known methods. The anti-tumor agents such as gemcitabine, carboplatin and paclitaxel show radiosensitizing effect and therefore, they can be used in a less dosage of the radiosensitizer of the present invention.

The dose of the radiosensitizer of the present invention varies depending on the age, weight, and sex of a patient, the administration method, and symptoms. In general, it is appropriate that the radiosensitizer is parenterally administered in an amount of 1 to 150 mg or orally administered in an amount of 10 to 250 mg as iron per day per adult. A patient may receive radiation generally within twelve hours, and desirably within one hour after the administration.

The $LD_{50}$ value in the case of single intravenous administration of chondroitin sulfate/iron colloid for intravenous injection to mice is 250 mg/kg or more as iron (Fujimoto et al., Yakugaku Zasshi vol.87, pp.677-681 (1967)), and the safety margin is wide.

Hereinafter, the present invention will be described in more detail by way of examples.

In the Examples, an albumin incorporating porphyrin-iron complex, saccharated ferric oxide and chondroitin sulfate/iron colloid are intraarterialy administered at a concentration of 1.7 mg/kg as iron to study a radiosensitizing effect.

EXAMPLES

2-[8-(2-methyl-1-imidazolyl)octanoyloxymethyl]-5,10,15,20-tetrakis{[α,α,α,α-o-(1-methylcyclohexanoylamino)]-phenyl}porphinatoiron complex to be used in the following examples was produced by the method described in T. Komatsu et al. , Bioconjugate Chemistry vol.13, pp.397-402 (2002).

Production Example 1

Production of albumin incorporating porphyrin-iron complex

Under a carbon monoxide atmosphere, 1. 5 L of an aqueous solution of 0.6 M L-ascorbic acid was added to 1.5 L of a solution of 1.07 mmol of 2-[8-(2-methyl-1-imidazolyl)octanoyloxymethyl]-5,10,15,20-tetrakis{[α,α,α,α-o-(1-methylcyclohexanoylamino)]-phenyl}porphinatoiron complex in ethanol for reduction, and the solution was added to 6.5 L of a phosphate buffered aqueous solution (pH 7.4, 1/30 mM) containing 0.27 mmol of recombinant human serum albumin (hereinafter referred to as rHSA), followed by stirring. Then, constant volume ultrafiltration dialysis was performed using an ultrafiltration device (ultrafiltration membrane manufactured by Millipore Corporation: molecular weight cut-off of 30, 000) while adding 60 L of a phosphate buffered aqueous solution (pH 7.4, 1/30 mM) to the mixture to thereby remove ethanol contained in the mixture. The mixture was concentrated to 300 mL, followed by adding aqueous phosphate buffer solution (pH7.4, 1/30 mM) so that the final concentration of rHSA is 0.75 mM (5w/v%), and a desired dispersion of an albumin incorporating porphyrin-iron complex (hereinafter abbreviated as rHSA-FecycP) was obtained.

The rHSA-FecycP was oxygenated by light-irradiation for 20 minutes using a 500W halogen lump under aeration with oxygen gas (100%), and the resultant was used in Example 1.

Experimental Example 1

Method: Tumor cells (Ascites hepatoma LY80) that had been subcultured in the abdominal cavity of a Donryu rat were implanted subfascially at the right thigh of the same strain rat in an amount of about $1.0 \times 10^6$ cells. Six days after the tumor implantation, ①5% (w/v%, hereinafter abbreviated as %) rHSA solution, ② rHSA-FecycP solution, a solution of saccharated ferric oxide, or ③a solution of chondroitin sulfate/iron colloid was administered to the common iliac artery of the rats at a concentration of 10 mL/kg (6 rats per group for ① to ③, and 3 rats per group for④). Note that the solution of rHSA-FecycP, the solution of saccharated ferric oxide, and the solution of chondroitin sulfate/iron colloid each were prepared to have a concentration of 3 mM as iron. After the administration, 20 Gy of X-rays were immediately irradiated to each tumor-implanted site. Meanwhile, for comparison, a non-treatment group and a rHSA-FecycP administration group both to which X-rays were not irradiated were provided (6 rats per group) .

The day of the tumor implantation was defined as day 0, and the life and death were observed up to 60 days after the implantation. The survival time of each group is shown by a box-and-whisker plot, and the logrank test (significance level of 5%) was performed as a significance test.

In the experiment, rHSA (25% preparation, produced by Bipha Co., Ltd. Japan), and as saccharated ferric oxide, Fesin (trade name, produced by Nippon Iyakuhin Kogyo Co. Ltd., now renamed Nichi-iko Pharmaceutical Co., Ltd.), and as chondroitin sulfate/iron colloid, Blutal (trade name, produced by Dainippon Pharmaceutical Co., Ltd., now renamed Dainippon Sumitomo Pharma Co., Ltd.) were used.

Results:

The obtained results are shown in Fig. 1.

Meanwhile, the major axes and minor axes of each tumor were measured on 6, 7, 9, 12, 15, 20, 25, 32, 39, 46, 53, and 60 days after the tumor implantation, and the tumor weights were calculated according to the following equation.

$$\text{Tumor weight (g)} = \text{major axis (mm)} \times \langle \text{minor axis (mm)} \rangle^2 \div 2 \div 1{,}000$$

The tumor weight of each group is shown as mean ± standard deviation, and the Tukey-Kramer multiple comparison test (significance level of 5%) was performed as a significance test. The obtained results are shown in Fig. 2.

The median of the survival time of the non-treatment group was found to be 21.5 days. Compared to the non-treatment group, the survival time of the rHSA-FecycP administration group (no irradiation) was not extended, but the survival times of all the groups to which X-rays had been irradiated were significantly extended. Meanwhile, compared to the irradiation group, the survival time of the rHSA administration + irradiation group was not extended, but the survival times of the rHSA-FecycP administration + irradiation group, the saccharated ferric oxide administration + irradiation group, and chondroitin sulfate/iron colloid administration + irradiation group were further significantly extended. Note that in the rHSA-FecycP administration + irradiation group and the saccharated ferric oxide administration + irradiation group, one case and two cases survived, respectively, even 60 days after the implantation.

The tumor weight of the non-treatment group increased with each passing day, while the weight of the rHSA-FecycP administration group (no irradiation) was also changed in the same way as the non-treatment group. Meanwhile, the weights of the groups to which X-rays had been irradiated were significantly decreased compared to the non-treatment group, respectively. There is no difference between the tumor weight of the rHSA administration + irradiation group and each of the tumor weights of the irradiation groups, but the tumor weights of the rHSA- FecycP administration + irradiation group, the saccharated ferric oxide administration + irradiation group, and chondroitin sulfate/iron colloid administration + irradiation group were significantly decreased. Note that in the rats of the rHSA-FecycP administration + irradiation group and the saccharated ferric oxide administration + irradiation group, that survived 60 days after the implantation, tumors were found to disappear 12 to 20 days after the implantation.

These results reveal that the rHSA-FecycP itself shows no antitumor effect, but use of it with X-rays enhances life-extending and tumor-growth delay effects by irradiation of X-rays. Meanwhile, these effects were observed also in saccharated ferric oxide and chondroitin sulfate/iron colloid, containing iron at the same concentration. Therefore, it became clear that iron which commonly exists in chemical structural formula participates in the enhancement, and an iron-containing compound is clearly useful as a radiosensitizer.

Production Example 2

Met-oxidation ($Fe^{2+} \rightarrow Fe^{3+}$) of albumin incorporating porphyrin-iron complex

The rHSA-FecycP was oxygenated by light-irradiation for 20 minutes using a 500W halogen lamp under aeration with oxygen gas (100%) and then incubated at 37°C for about 5 days, followed by autoxidation, to thereby yield met-oxidized ($Fe^{2+} \rightarrow Fe^{3+}$, see Emil L. Smith et al., Principles of Biochemistry-Mammalian Biochemistry, seventh edition pp.668-689, Hirokawa Publishing Co., Tokyo (1990)) rHSA-FecycP (hereinafter abbreviated as Met-rHSA-FecycP). Note that the met-oxidation of the rHSA-FecycP was confirmed by reduction of the absorbance at λmax 539.0 nm.

Experimental Example 2

Method) Tumor cells (Ascites hepatoma LY80) that had been subcultured in the abdominal cavity of a Donryu rat were implanted subfascially at the right thigh of the same strain rat in an amount of about $1.0 \times 10^6$ cells. Six days after the tumor implantation, 5% rHSA or Met-rHSA-FecycP was administered to the common iliac artery of the rats at a concentration of 10 mL/kg (6 rats per group) . Note that the Met-rHSA-FecycP was prepared to have a concentration of 3 mM as an iron. After the administration, 20 Gy of X-rays were immediately irradiated to each tumor-implanted site. Meanwhile, for comparison, a non-treatment group and a Met-rHSA-FecycP group both to which X-rays were not irradiated were provided (6 rats per group).

The day of the tumor implantation was defined as day 0, and the major axes and minor axes of tumors were measured on 6, 7, 9, 12, 15, 20, 25, 32, 39, 46, 53, and 60 days after the tumor implantation and the tumor weights were calculated according to the following equation.

$$\text{Tumor weight (g)} = \text{major axis (mm)} \times \langle\text{minor axis (mm)}\rangle^2 \div 2 \div 1{,}000$$

The tumor weight of each group is shown as mean ± standard deviation, and the Tukey-Kramer multiple comparison test (significance level of 5%) was performed as a significance test. The obtained results are shown in Fig. 3.

The tumor weight of the non-treatment group increased with each passing day, while the tumor weight of the Met-rHSA-FecycP administration group (no irradiation) was also changed in the same way as the non-treatment group.

Meanwhile, the tumor weights of the groups to which X-rays had been irradiated were significantly decreased. Compared to the rHSA administration + irradiation group, the tumor weight of the Met-rHSA-FecycP administration + irradiation group was significantly decreased. Note that in the Met-rHSA-FecycP administration + irradiation group, one case survived even 60 days after the implantation. In the case, the tumor disappeared 15 days after the implantation. Therefore, it became clear that the Met-rHSA-FecycP enhances tumor-growth delay effects by irradiation of X-rays to tumor tissues.

These results reveal that use of the Met-rHSA-FecycP with X-rays enhances tumor-growth delay effects by irradiation of X-rays, like the rHSA-FecycP and saccharated ferric oxide each containing iron at the same concentration. Therefore, it became clear that both Fe (II) (valence of iron in the rHSA-FecycP) and Fe (III) (valence of iron in saccharated ferric oxide and the Met-rHSA-FecycP) in the chemical structural formula have the enhancement effect, and an iron-containing compound is clearly useful as a radiosensitizer.

INDUSTRIAL APPLICABILITY

The radiosensitizer of the present invention has a high radiosensitizing effect, low toxicity, and is stable. Thus, the radiosensitizer of the present invention is useful as a radiosensitizer in chemotherapy for cancer.

## Claims

1. A radiosensitizer which comprises an iron compound.

2. The radiosensitizer according to claim 1, wherein the iron compound is an inorganoiron compound, or an organoiron compound selected from the group consisting of saccharated ferric oxide, iron citrate, ironacetate, irongluconate, chondroitin sulfate/iron colloid, cideferron, ferrotrenine, iron fumarate, iron pyrophosphate, a porphyrin-iron complex, and an albumin incorporating porphyrin-iron complex.

3. The radiosensitizer according to claim 2, wherein the inorganoiron compound is selected from the group consisting of iron chloride, iron oxide, iron hydroxide, and iron sulfate.

4. The radiosensitizer according to claim 1, wherein the iron compound is an organoiron compound selected from the group consisting of saccharated ferric oxide, iron citrate, iron gluconate, chondroitin sulfate/iron colloid, cideferron, ferrotrenine, iron fumarate, iron pyrophosphate, a porphyrin-iron complex, and an albumin incorporating porphyrin-iron complex.

5. The radiosensitizer according to claim 1, wherein the iron compound is saccharated ferric oxide, chondroitin sulfate/ iron colloid, a porphyrin-iron complex, or an albumin incorporating porphyrin-iron complex.

6. The radiosensitizer according to claim 4 or 5, wherein iron in the porphyrin-iron complex is Fe(II).

7. The radiosensitizer according to claim 4 or 5, wherein the porphyrin-iron complex has not a phenyl substituent substituted by a substituent having a boron atom on the porphyrin ring of the porphyrin-iron complex.

8. The radiosensitizer according to claim 4 or 5, wherein the porphyrin-iron complex is a non-boronated porphyrin-iron complex.

9. The radiosensitizer according to claim 1, wherein the iron compound is an albumin incorporating porphyrin-iron complex.

10. The radiosensitizer according to claim 9, wherein the albumin is recombinant albumin.

11. A kit for treatment of cancer containing an iron compound as a radiosensitizer, and a chemotherapeutic agent.

12. The kit as claimed in claim 11, wherein the radiosensitizer and the chemotherapeutic agent are separately packaged.

13. The kit as claimed in claim 11, **characterized in that** the radiosensitizer as defined in claim 1 is administered within one hour after a chemotherapeutic agent is administered, followed by radiation treatment.

14. Use of an iron compound as defined in claims 1 to 10 for the preparation of a radiosensitizer and a kit for treatment

of cancer containing said iron compound as a radiosensitizer, and a chemotherapeutic agent.

**15.** The use as claimed in claim 14, wherein the radiosensitizer and the chemotherapeutic agent are separately packaged.

**16.** The use as claimed in claim 14, **characterized in that** the radiosensitizer as defined in claim 1 is administered within one hour after a chemotherapeutic agent is administered, followed by radiation treatment.

Fig.1

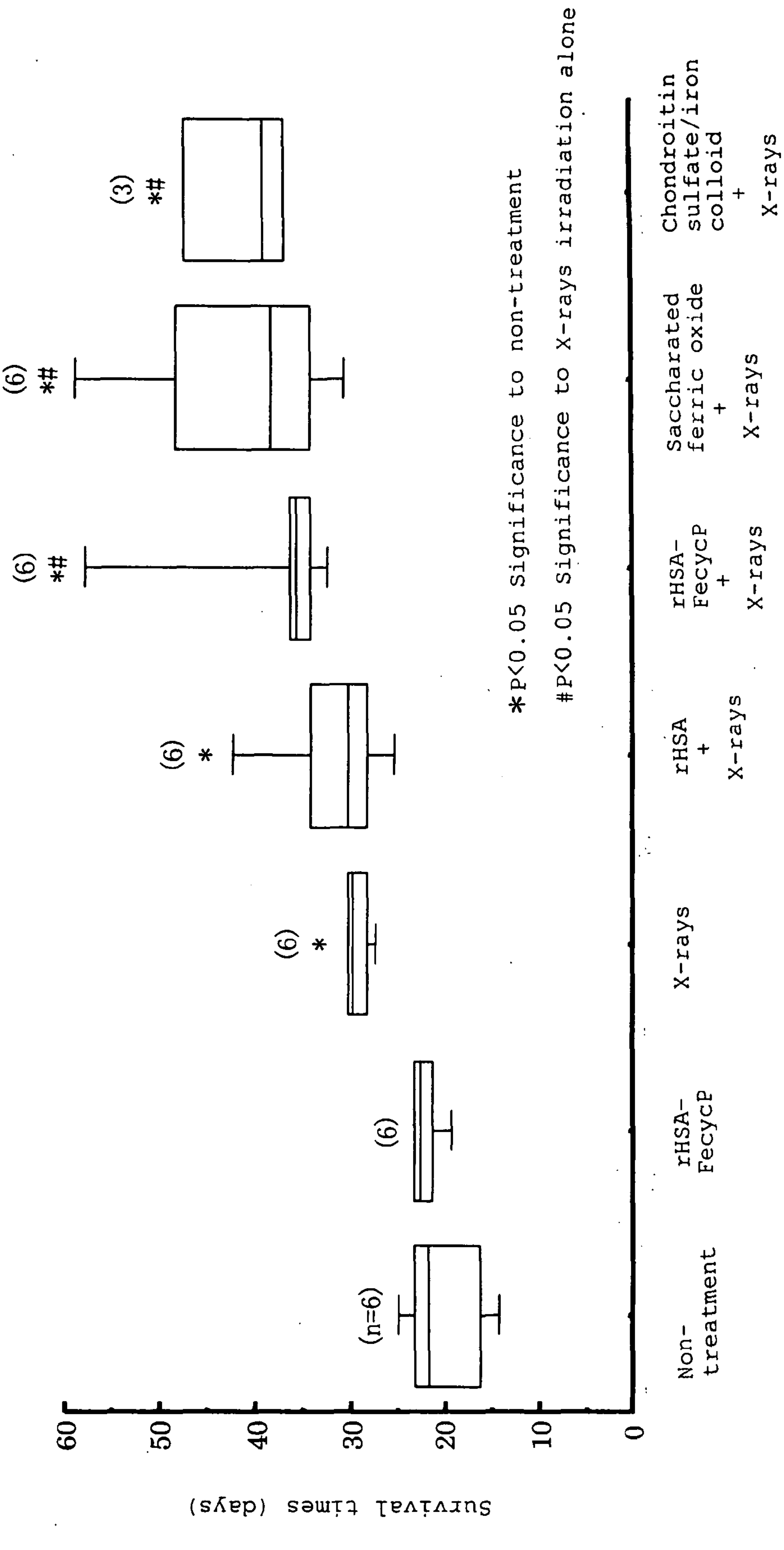

Survival times (days)
0
10
20
30
40
50
60
(n=6)
(6)
(6)
*
(6)
*
(6)
*#
(6)
*#
(3)
*#
*P<0.05 Significance to non-treatment
#P<0.05 Significance to X-rays irradiation alone
Non-treatment
rHSA-FecycP
X-rays
rHSA + X-rays
rHSA-FecycP + X-rays
Saccharated ferric oxide + X-rays
Chondroitin sulfate/iron colloid + X-rays

Fig.2

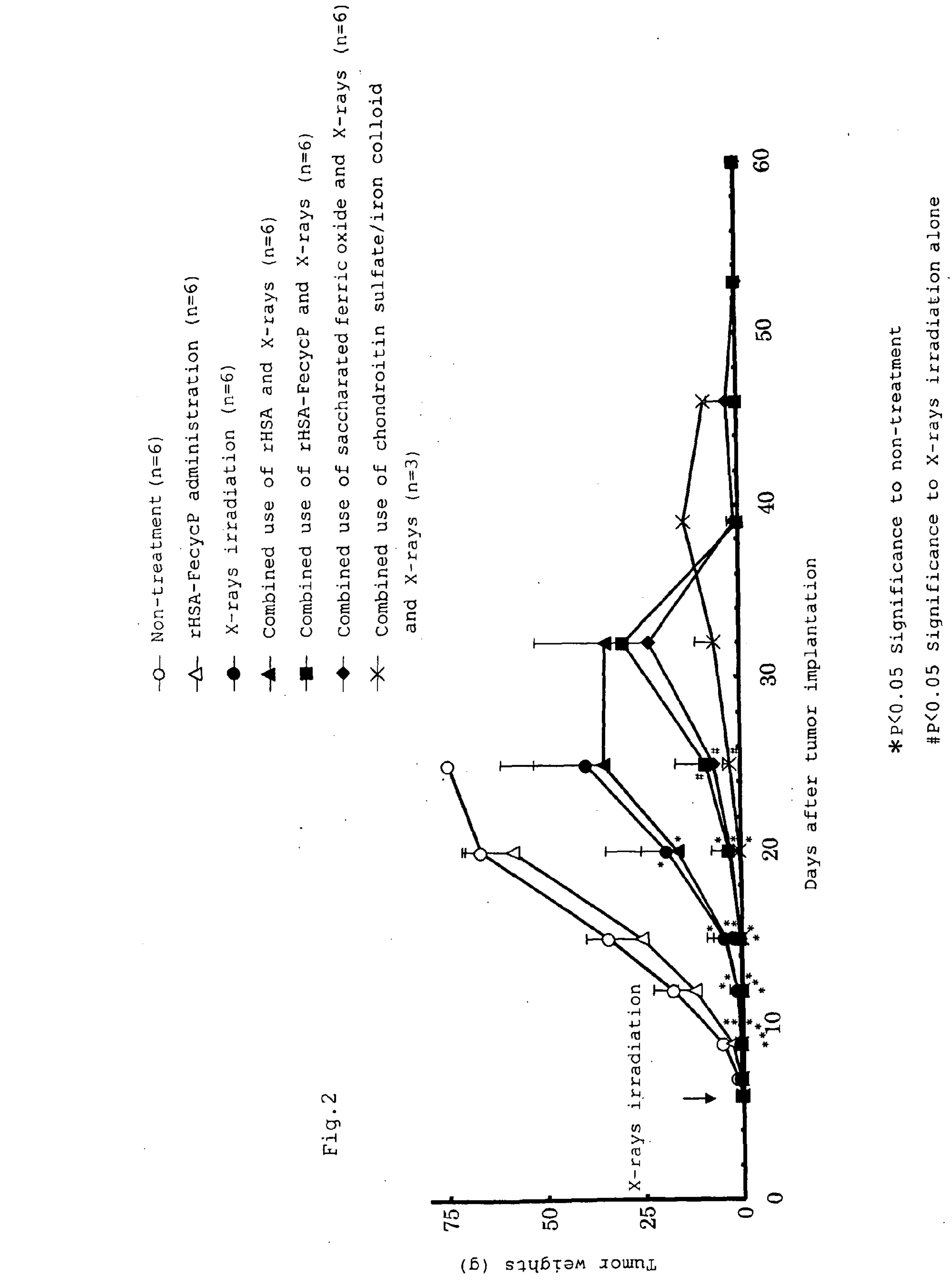
Non-treatment (n=6)
rHSA-FecycP administration (n=6)
X-rays irradiation (n=6)
Combined use of rHSA and X-rays (n=6)
Combined use of rHSA-FecycP and X-rays (n=6)
Combined use of saccharated ferric oxide and X-rays (n=6)
Combined use of chondroitin sulfate/iron colloid and X-rays (n=3)
X-rays irradiation
Tumor weights (g)
75
50
25
0
0
10
20
30
40
50
60
Days after tumor implantation
*P<0.05 Significance to non-treatment
#P<0.05 Significance to X-rays irradiation alone

Fig.3

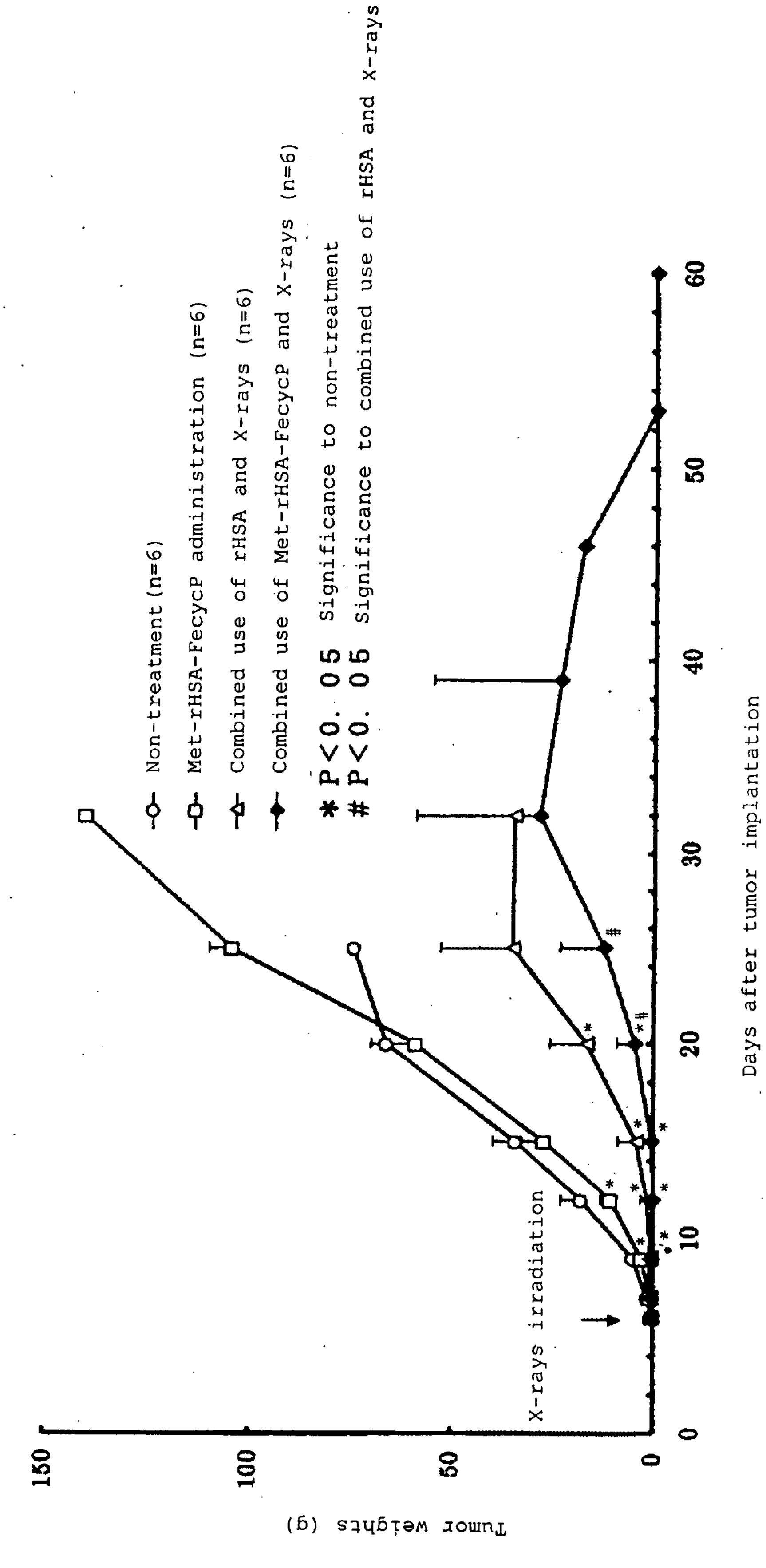
Non-treatment (n=6)
Met-rHSA-FecycP administration (n=6)
Combined use of rHSA and X-rays (n=6)
Combined use of Met-rHSA-FecycP and X-rays (n=6)
* P<0. 05 Significance to non-treatment
# P<0. 05 Significance to combined use of rHSA and X-rays
X-rays irradiation
Tumor weights (g)
Days after tumor implantation
0
50
100
150
0
10
20
30
40
50
60

European Patent Office

# EUROPEAN SEARCH REPORT

Application Number
EP 06 00 1402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 512 399 A (NIPRO CORPORATION) 9 March 2005 (2005-03-09) * page 2, lines 53,54 * * page 2, line 58 - page 3, line 3 * * page 3, lines 13,14,43-45 * * page 3, line 57 - page 4, line 1 * * page 6, line 7 * * paragraph [0030] * * examples * See formula i, ii ----- | 1,2,4-10 | A61K41/00 A61K47/48 A61K31/409 A61P35/00 |
| X | US 4 727 068 A (ABRAMS ET AL) 23 February 1988 (1988-02-23) * column 1, lines 1-4,15-19 * * column 1, lines 23-25,57-62 * * column 2, lines 58-61 * * column 3, lines 11-17 * * example 2 * * claims 1-4 * ----- | 1 | |
| X | WO 02/098417 A (BROOKHAVEN SCIENCE ASSOCIATES; MIURA, MICHIKO; SLATKIN, DANIEL, N) 12 December 2002 (2002-12-12) * claims 1,3,11,16 * * page 8, lines 12-15 * * page 9, line 7 * * page 10, line 11 * ----- | 1,2,4,5, 7,8 | TECHNICAL FIELDS SEARCHED (IPC) A61K |
| X | US 2003/083494 A1 (MIURA MICHIKO ET AL) 1 May 2003 (2003-05-01) * paragraphs [0003], [0004] * * paragraphs [0019] - [0021] * * claims 1,3,8,16,23,30 * See aforementioned passages: iron (Fe) ----- -/-- | 1,2,4,6 | |

The present search report has been drawn up for all claims

| Place of search | Date of completion of the search | Examiner |
|---|---|---|
| Munich | 7 March 2006 | Veronese, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 00 1402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 375 634 B1 (CARROLL ROBERT G) 23 April 2002 (2002-04-23) * column 22, line 60 - column 23, line 10 * * column 24, line 65 - column 26, line 2 * ----- | 1 | |
| X | US 5 776 925 A (YOUNG ET AL) 7 July 1998 (1998-07-07) * claims 4,13,14 * * column 6, lines 13-34 * * column 3, lines 28-34 * ----- | 1,11-16 | |
| P,X | EP 1 538 151 A (NIPRO CORPORATION) 8 June 2005 (2005-06-08) * claims; figure 2 * ----- | 1,2,4-10 | |
| X | TEICHER B A ET AL: "Some complexes of cobalt(III) and iron(III) are radiosensitizers of hypoxic EMT6 cells." RADIATION RESEARCH. JAN 1987, vol. 109, no. 1, January 1987 (1987-01), pages 36-46, XP008060964 ISSN: 0033-7587 * abstract * * page 37, paragraphs 2,3 * * pages 42-44 * ----- | 1 | TECHNICAL FIELDS SEARCHED (IPC) |
| X | JOY A M ET AL: "High efficiency of ferricenium salts as radiosensitizers of V79 cells in vitro and the KHT tumor in vivo." INTERNATIONAL JOURNAL OF RADIATION ONCOLOGY, BIOLOGY, PHYSICS. APR 1989, vol. 16, no. 4, April 1989 (1989-04), pages 1053-1056, XP008061010 ISSN: 0360-3016 * the whole document * ----- -/-- | 1 | |

The present search report has been drawn up for all claims

| Place of search | Date of completion of the search | Examiner |
|---|---|---|
| Munich | 7 March 2006 | Veronese, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

7

European Patent Office

# EUROPEAN SEARCH REPORT

Application Number
EP 06 00 1402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE BIOSIS [Online] BIOSCIENCES INFORMATION SERVICE, PHILADELPHIA, PA, US; 1980, DOUPLE E B ET AL: "POTENTIATION OF CELLULAR RADIO SENSITIVITY BY NITROPRUSSIDE AND VITAMIN B-12" XP002370388 Database accession no. PREV198172075102 * abstract * & INTERNATIONAL JOURNAL OF RADIATION ONCOLOGY, BIOLOGY, PHYSICS, vol. 6, no. 11, 1980, pages 1545-1550, ISSN: 0360-3016 ----- | 1 | |
| X | DATABASE MEDLINE [Online] US NATIONAL LIBRARY OF MEDICINE (NLM), BETHESDA, MD, US; December 1996 (1996-12), VEROVSKI V N ET AL: "Intrinsic radiosensitivity of human pancreatic tumour cells and the radiosensitising potency of the nitric oxide donor sodium nitroprusside." XP002370389 Database accession no. NLM8956786 * abstract * & BRITISH JOURNAL OF CANCER. DEC 1996, vol. 74, no. 11, December 1996 (1996-12), pages 1734-1742, ISSN: 0007-0920 ----- | 1 | TECHNICAL FIELDS SEARCHED (IPC) |

The present search report has been drawn up for all claims

| Place of search | Date of completion of the search | Examiner |
|---|---|---|
| Munich | 7 March 2006 | Veronese, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

7

EPO FORM 1503 03.82 (P04C01)

## ANNEX TO THE EUROPEAN SEARCH REPORT ON EUROPEAN PATENT APPLICATION NO. EP 06 00 1402

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2006

| Patent document cited in search report | | Publication date | Patent family member(s) | | | Publication date |
|---|---|---|---|---|---|---|
| EP 1512399 | A | 09-03-2005 | AU | 2003241909 | A1 | 19-12-2003 |
| | | | CA | 2487757 | A1 | 11-12-2003 |
| | | | CN | 1674891 | A | 28-09-2005 |
| | | | WO | 03101451 | A1 | 11-12-2003 |
| | | | JP | 2004010495 | A | 15-01-2004 |
| | | | US | 2005222116 | A1 | 06-10-2005 |
| US 4727068 | A | 23-02-1988 | AU | 584048 | B2 | 11-05-1989 |
| | | | AU | 6435486 | A | 30-04-1987 |
| | | | CA | 1277912 | C | 18-12-1990 |
| | | | DE | 3683612 | D1 | 05-03-1992 |
| | | | DK | 508486 | A | 24-04-1987 |
| | | | EP | 0227234 | A1 | 01-07-1987 |
| | | | JP | 62174014 | A | 30-07-1987 |
| | | | NO | 864228 | A | 24-04-1987 |
| | | | NZ | 218020 | A | 27-09-1989 |
| | | | ZA | 8607862 | A | 28-10-1987 |
| WO 02098417 | A | 12-12-2002 | CA | 2449316 | A1 | 12-12-2002 |
| | | | CN | 1620289 | A | 25-05-2005 |
| | | | EP | 1404319 | A1 | 07-04-2004 |
| | | | GB | 2393123 | A | 24-03-2004 |
| | | | JP | 2005504012 | T | 10-02-2005 |
| US 2003083494 | A1 | 01-05-2003 | US | 2003165426 | A1 | 04-09-2003 |
| | | | US | 2003032799 | A1 | 13-02-2003 |
| | | | ZA | 200400056 | A | 14-09-2004 |
| US 6375634 | B1 | 23-04-2002 | NONE | | | |
| US 5776925 | A | 07-07-1998 | AT | 277637 | T | 15-10-2004 |
| | | | AU | 715025 | B2 | 13-01-2000 |
| | | | AU | 1833397 | A | 20-08-1997 |
| | | | CN | 1213313 | A | 07-04-1999 |
| | | | DE | 69730961 | D1 | 04-11-2004 |
| | | | DE | 69730961 | T2 | 24-11-2005 |
| | | | EP | 0912196 | A1 | 06-05-1999 |
| | | | ES | 2227671 | T3 | 01-04-2005 |
| | | | HK | 1018588 | A1 | 25-02-2005 |
| | | | JP | 2000503989 | T | 04-04-2000 |
| | | | NO | 983429 | A | 24-09-1998 |
| | | | NZ | 331513 | A | 26-10-2001 |
| | | | WO | 9726915 | A1 | 31-07-1997 |
| EP 1538151 | A | 08-06-2005 | US | 2005085410 | A1 | 21-04-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9510307 A
- JP 5194268 A
- JP 2000212087 A
- JP 6298739 A
- WO 02098417 A
- JP 2004277329 A
- JP 2004010495 A
- JP 6271577 A


### Non-patent literature cited in the description


- **C. J. HALFMAN ; T. NISHIDA.** *Biochemistry,* 1972, vol. 11, 3493
- **T.KOMATSU et al.** *Bioconjugate Chemistry,* 2002, vol. 13, 397-402
- **T.KOMOTSU et al.** *Bioconjugate Chemistry,* 2002, vol. 13, 397-402
- **K. KOBAYASHI et al.** *Therapeutic Apheresis,* 1998, vol. 2 (4), 257-262
- **FUJIMOTO et al.** *Yakugaku Zasshi,* 1967, vol. 87, 677-681
- **T. KOMATSU et al.** *Bioconjugate Chemistry,* 2002, vol. 13, 397-402
- **EMIL L. SMITH et al.** Principles of Biochemistry-Mammalian Biochemistry. Hirokawa Publishing Co, 1990, 668-689